# EUROPEAN PATENT SPECIFICATION

(11) **EP 1 356 122 B1**
(45) Date of publication and mention of the grant of the patent: **25.03.2009**
(21) Application number: 02716685.9
(22) Date of filing: 25.01.2002
(51) Int. Cl.: C12Q 1/68, C12N 15/10

(54) **AMMONIUM SULFATE FOR NEUTRALIZATION OF INHIBITORY EFFECTS**
AMMONIUMSULFAT ZUR AUFHEBUNG VON INHIBITORISCHEN EFFEKTEN
SULFATE D'AMMONIUM POUR NEUTRALISER DES EFFETS INHIBITEURS

(30) Priority: 25.01.2001 US 264508 P; 26.01.2001 US 264488 P
(43) Date of publication of application: 29.10.2003
(73) Proprietor: QIAGEN GmbH, 40724 Hilden (DE)
(72) Inventor: KORFHAGE, Christian, 40764 Langenfeld (DE); WYRICH, Ralf, 41542 Dormagen (DE); OELMÜLLER, Uwe, 40699 Erkrath (DE)
(86) International application number: PCT/EP2002/000763
(87) International publication number: WO 2002/059360

(56) References cited:
- WO-A-00/06780

## Description

### TECHNICAL FIELD

The present invention relates to techniques in biochemistry, more particularly to techniques for ribonucleic acid purification.

### BACKGROUND OF THE INVENTION

It is well known that a lot of molecules such as proteins, spermine, spermidine, cationic detergents, ethidium bromide, SybrGreen^{™} dye, actinomycin etc. are able to bind to and to inhibit the function and analysis of RNA. The binding mode of most inhibitory substances to RNA is ionic, ionic stabilized by hydrophilic interaction, or ionic stabilized by lipophilic interaction. In many cases the interaction between molecules and the RNA ligand is very strong so that very harsh conditions (*e.g*., denaturating agents, chaotropic agents, detergents, phenol etc.) are needed to diminish the interaction between RNA and inhibitory molecules. In some cases even harsh conditions do not stop the interaction. In other cases, the harsh conditions interfere with downstream applications of the RNA. Accordingly, a method is needed which mitigates the interaction of inhibitory molecules to RNA, but does not interfere with the function and analysis of RNA.

WO-A-00/06780 relates to a novel method and reagents for preserving the RNA in tissue fragments at temperatures above the freezing point of the preservatives for extended periods of time, including days to months, prior to RNA isolation, whereas the present invention relates to a method to neutralize the inhibitory effect of an agent that binds to, or cleaves RNA on RNA isolated from a natural source or artificially synthesized.

It is also well known that nucleophilic agents like the anion OH⁻ or the 2'-OH group of the RNA-ribose, in presence of a catalyst and/or bivalent cations, can serve to cleave RNA. Cleavage of RNA interferes with the function and analysis of the RNA molecule. Neutralization of cleaving agents often is only possible by purification. A method is needed whereby the cleavage effects of various molecules are inhibited.

### SUMMARY OF THE INVENTION

The invention describes the addition of (NH₄)₂SO₄ to an environment containing RNA. The final concentration is below 20 g/100 ml (1.51 M). The addition of (NH₄)₂SO₄ to the environment neutralizes the inhibitory effects of agents that bind to, or cleave, RNA. Such agents include cationic detergents (*e.g.*, CATRIMOX, and cetyltrimethylammonium bromide = CAB. *See, e.g.,* European Patent Application EP 1031626 A1), proteins, ethidium bromide, SybrGreen™ dye, polyamines (*e.g*., spermine, spermidine, putresceine etc.), charged polysaccharides, glycoproteins, nucleophiles, bases etc. With the presence of (NH₄)₂SO₄ within the environment, the inhibitory or cleaving properties of agents binding to RNA does not interfere with the function and analysis of the environment or the RNA contained therein.

As such, the present invention relates to a method to neutralize the inhibitory or destructive effect of an agent that binds to, or cleaves RNA on RNA isolated from a natural source or artificially synthesized, comprising adding ammonium sulfate to a composition comprising said RNA and said agent, where the final concentration of ammonium sulfate in the composition is below 20g / 100 ml.

In one aspect, the present invention provides a method of RNA purification comprising adding ammonium sulfate to a composition comprising RNA, where the final concentration of ammonium sulfate in the composition is below 20 g / 100 mL.

In another aspect, the present invention provides a method of RNA purification comprising adding ammonium sulfate to a composition comprising RNA, where the final concentration of ammonium sulfate in the composition is about 1-64 mM. In various other aspects, the final concentration of ammonium sulfate in the composition is about 5-32 mM, or is about 10 mM.

In another aspect, the present invention provides a method of RNA purification comprising adding ammonium sulfate to a composition comprising RNA, where the composition also comprises one or more agents that bind to and then inhibit and/or cleave the RNA. The ammonium sulfate is added to this composition in an amount effective to reduce the detrimental effects of the agent(s) on RNA activity. Typically, this amount is 20 g ammonium sulfate per 100 mL of RNA-containing composition.

In any of the aforesaid aspects, in an additional aspect the composition may further comprise a contaminant selected from RNA binding agents. In any of the aforesaid aspects, in an addition aspect the composition may further comprise a polyamine as a contaminant, where spermine, spermidine, and putresceine are exemplary polyamine contaminants. In any of the aforesaid aspects, in an additional aspect the composition may further comprise a cationic detergent as a contaminant In any of the aforesaid aspects, in an additional aspect the composition may further comprise a nucleic acid dye as a contaminant, where ethidium bromide and SybrGreen™ dye are exemplary nucleic acid dye contaminants. In any of the aforesaid aspects, in an additional aspect the composition may further comprise actinomycin as a contaminant. In any of the aforesaid aspects, in an additional aspect, the composition may further comprise a charged polysaccharide as a contaminant. In any of the aforesaid aspects, in an additional aspect, the composition may further comprise glycoprotein as a contaminant. In any of the aforesaid aspects, in an additional aspect the composition may further comprise a nucleophile as a contaminant. In additional aspects, the present invention provides that the composition to which ammonium sulfate is added may contain any two or more of the specifically enumerated contaminants, i.e., any two or more (*e.g*., three, four) of RNA binding agent, polyamine, cationic detergent, nucleic acid dye, actinomycin, charged polysaccharide, glycoprotein, and nucleophile.

### BRIEF DESCRIPTION OF THE DRAWINGS

Figure 1: Inhibitory effect of cationic detergent during reverse-transcriptase reaction.
Figure 2: Reverse transcription of RNA that contained cationic detergents is inhibited by cationic detergent. Addition of (NH₄)₂SO₄ to the RNA inhibited by cationic detergents neutralize the inhibitory effect and shows a high increase in RT performance.
Figure 3: Pure RNA without cationic detergents showed only a very low increase in RT-performance.
Figure 4: Analysis of RNAs prepared by a classical method without cationic detergent or prepared by cationic detergent. The RNA prepared with cationic detergent showed increased threshold-cycle. Elution of RNA prepared with cationic detergent with a 10 mM (NH₄)₂SO₄ solution and denaturation at 65°C neutralize the inhibitory effect of cationic detergent. The threshold-cycle of each bar reflects 6 independent values.
Figure 5: Stability of the neutralization effect during time RNA prepared by classical method [no inhibitors](C) or by cationic detergent with elution in water (B) or with elution in 5 mM (NH₄)₂SO₄ (A). The GAPDH PDAR of Applied Biosystem was used for the TaqMan RT-PCR. The Figure summarizes the analyses of three independent blood donors.
Figure 6: Stability of the neutralization effect during time RNA prepared by classical method [no inhibitors] (C) or by cationic detergent with elution in water (B) or with elution in 5 mM (NH₄)₂SO₄ (A). The TNFalpha PDAR of Applied Biosystem was used for the TaqMan RT-PCR. The Figure summarizes the analyses of three independent blood donors.
Figure 7: Inhibitory effect of spermine during reverse-transcriptase reaction.
Figure 8: Reverse transcription of RNA that contained spermine is inhibited by binding of spermine to RNA (lanes 2). Addition of (NH₄)₂SO₄ to the RNA inhibited by spermine neutralize the inhibitory effect (lanes 3). Lane 1 serves as a control that is not inhibited by spermine.
Figure 9: Inhibitory effect of SybrGreen™ dye during reverse-transcriptase reaction. Final concentration during reverse-transcriptase reaction is indicated. PCR was performed to quantify cDNA-synthesis.
Figure 10: Reverse transcription of RNA that contained SybrGreen™ dye is inhibited by binding of SybrGreen™ dye to RNA (lanes 2). Addition of (NH₄)₂SO₄ to the RNA inhibited by spermine neutralize the inhibitory effect (lanes 1). Lane 3 serves as a control that is not inhibited by SybrGreen™ dye.
Figure 11: Tight junction of (NH₄)₂SO₄ ions to RNA is indicated by reduced ethidium bromide binding to the RNA backbone and less electrophoretic mobility which indicates a strong isolation of the negative charged RNA backbone.
Figure 12: Hypochromic effect of RNA at 260 nM due to the addition of (NH₄)₂SO₄.
Figure 13: Incubation of RNA at different pHs indicated and 10 mM (NH₄)₂SO₄ for one hour at 37°C.
Figure 14: Relative activities of reverse transcriptase using RNA dissolved in solutions of different (NH₄)₂SO₄ concentrations.

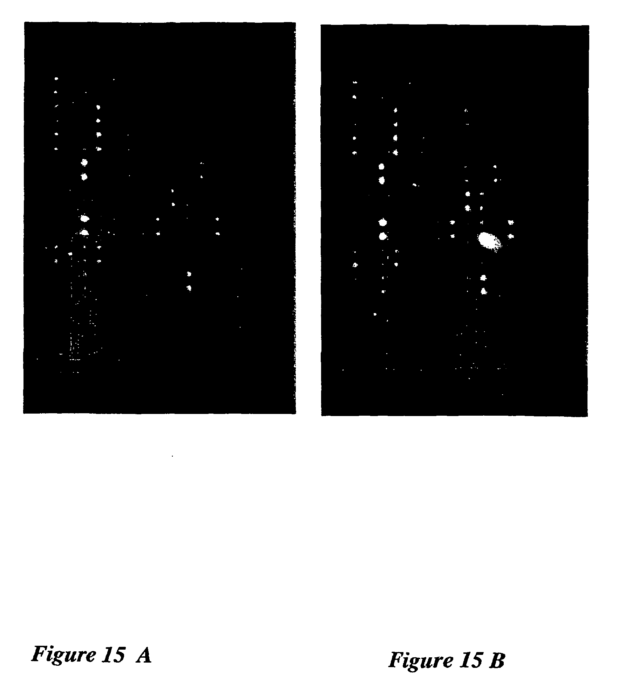

Figure 15: Denaturation of RNA in a (NH₄)₂SO₄ containing solution prior labeling reaction more signals are visible and higher signal-intensities were obtained (B) compared to the standard labeling procedure without denaturation in a (NH₄)₂SO₄ containing solution (A).

### DETAILED DESCRIPTION OF THE INVENTION

Ribonucleic acid (RNA) is a substance synthesized biologically and synthetically. RNA serves many functions as information molecule, reaction substrate, reaction catalyst, recognition element, structural element etc. For most analysis methods and functions concerning RNA itself or RNA as an analysis tool the purity of RNA is important. That means that *e.g.,* other molecules which bind the RNA are able to inhibit the analysis/function of/using the RNA molecule or destroy the structure of the RNA molecule. For this reason it is important to eliminate the inhibitory or destroying function of molecules binding to the RNA. Moreover stable secondary structures of RNA often results in reduced function and analysis quality. Secondary structure depends on the environment of the RNA molecule. The present invention features the addition of ammonium sulfate (NH₄)₂SO₄ to RNA in solution to eliminate the inhibitory or destroying effect of molecules binding to the RNA to be taken as information molecule, reaction substrate, reaction catalyst, recognition element, structural element etc. Furthermore (NH₄)₂SO₄ solves secondary structures of RNA to make RNA more accessible to reactions and analysis.

The present invention is directed to the addition of (NH₄)₂SO₄ to an environment containing RNA. In one aspect, the final concentration of the (NH₄)₂SO₄ is below 20 g/100 mL (1.51 M). The addition of (NH₄)₂SO₄ to the environment neutralizes the inhibitory effects of agents that bind to, or cleave, the RNA. Such agents include cationic detergents (*e.g*., CATRIMOX and cetyltrimethylammonium bromide=CTAB. See, *e.g*., European Patent Application EP 1031626 A1), proteins, ethidium bromide, SybrGreen™ dye, polyamines (*e.g*., spermine, spermidine, putresceine etc.), charged polysaccharides, glycoproteins, nucleophiles, bases etc. With the presence of (NH4)₂SO₄ in the environment, the inhibitory or cleaving properties of agents binding to RNA do not interfere with the function and analysis of the environment or the RNA contained therein.

In another aspect, the present invention provides a method of RNA purification comprising adding ammonium sulfate to a composition comprising RNA, where the composition also comprises one or more agents that bind to and then inhibit and/or cleave the RNA. The ammonium sulfate is added to this composition in an amount effective to reduce the detrimental effects of the agent(s) on RNA activity. Typically, this amount is 20 g ammonium sulfate per 100 mL of RNA-containing composition. Before describing the present invention in more detail, some definitions are provided.

### Definitions:

### Ribonucleic acid (RNAs):

The RNA is defined as any ribonucleic acid of interest, known or unknown to the practitioner. The RNA may be isolated from natural source or artificially synthesized in chemical or enzymatic reactions. The RNA may contain unmodified or modified nucleotides. The RNA may be untagged or tagged by a known or unknown tag.

### Function of RNA:

Function of RNA is defined as any *in-vivo* or *in-vitro* function that RNA can have, including, without limitation, coding function (*e.g*., genomic RNA), enzymatic function (*e.g*., ribozyme function), binding function (*e.g*., hybridizing function, aptamer function etc.), template function, substrate function, structural function, sensor function, and the like.

### Analysis of RNA:

Analysis of RNA can be performed by any biological, biochemical, biophysical, chemical or physical (*e.g*., mass spectrometry) analysis method to determine any property of the RNA to be analyzed. Exemplary properties include, without limitation, size, structure, charge, pH, modification, amount, number, solubility, stability, concentration, and the like.

Often used biological analysis methods include *in-vivo* methods in any organism like viruses, phages, archae-bacteria, bacteria, fungi, plants, animals and extra-terrestic organisms.

Often used biochemical analysis methods include *in-vitro* methods *e.g*., hybridization, RT-reaction, RT-PCR, RT- and RT-PCR mediated analysis methods, sequencing, linear and exponential isothermal amplification reactions (NASBA, TMA, 3SR), Ligase Chain reaction (LCA), Oligonucleotide Ligase Assay (OLA), Invader™, Branched DNA, primer extension assays, protection assays, binding assays, function assays, etc.

Often used chemical, biophysical and physical analysis methods include any type of spectrometry, chromatography, crystallization, ionization, photometry, etc.

### Primer:

Oligonucleotide primers useful in the analysis of RNA may be any oligonucleotide of two or more nucleotides in length. Oligonucleotide primers are used to hybridize to a region of a target nucleic acid to analyze the target nucleic acid or to facilitate the polymerization of a complementary nucleic acid.

### Reverse Transcriptase-reaction (RT-reaction):

RT-reactions (cDNA-synthesis reactions) are oligonucleotide-primer-dependent DNA-synthesis reactions. Specific primed reactions for specific and efficient cDNA-synthesis using DNA-polymerases can be performed using RNA-directed and/or DNA-directed DNA-polymerases wherein cDNA-synthesis comprises two steps: (a) the 1^{st}-strand cDNA-synthesis, which is sufficient for a lot of application (*e.g*., RT-PCR, RT-PCR dependent processes etc.); and (b) 2^{nd}-strand synthesis, which is needed for most cloning purposes.

cDNA-synthesis comprises the steps:
(1) for 1^{st}-strand cDNA-synthesis the synthesis of a DNA first-strand complementary to the target nucleic acid is performed by hybridizing a oligonucleotide-primer P1 to a complementary sequence of the target nucleic acid followed by elongation of oligonucleotide-primer P1 using deoxyribonucleotides and a RNA-directed or DNA-directed DNA-polymerase, wherein said target nucleic acid is deoxyribonucleic acid (RNA);
(2) for 2^{nd}-strand cDNA synthesis the synthesis of a DNA second-strand complementary to the DNA first-strand can be performed by enzymatic, chemical or thermal removal of the target nucleic acid from DNA first-strand followed by hybridizing a second oligonucleotide-primer P2 to a complementary sequence of the DNA first-strand or by using a hairpin-loop of the DNA first-strand followed by elongation of said oligonucleotide-primer P2 or said hairpin-loop of the DNA first-strand using deoxyribonucleotides and a DNA-polymerase wherein DNA second-strand is complementary to DNA first-strand;
(3) wherein said oligonucleotide-primer P1 bear a sequence at its 3'-end hybridizing to the target nucleic acid;
(4) wherein said oligonucleotide-primer P2 bear a sequence at its 3'-end hybridizing to the DNA first-strand;
(5) wherein said oligonucleotide-primer P1 and P2 may bear a another functional non-hybridizing sequence at their 5'-ends.

### Reverse Transcriptase-Polymerase-chain-reaction (RT-PCR):

In RT-PCR, the reaction is bipartite and contains a RT-reaction and a PCR. Both reactions (RT-reaction and PCR) can be performed sequentially in two independent set-ups (two-step RT-PCR) or in one set-up (one-step RT-PCR).

For RT-PCR (one-step as well as two-step RT-PCR) only 1^{st}-strand cDNA-synthesis is performed during the RT-reaction. 1^{st}-strand cDNA is used for the amplification step during PCR that usually comprises the steps:
(1) initial denaturation of the target nucleic acid
(2) sequence dependent hybridization of primer P2 to a complementary sequence
(3) elongation of primer P2 using deoxyribonucleotides and a DNA-polymerase wherein synthesized DNA is complementary to cDNA first-strand;
(4) denaturation of generated double-strand
(5) sequence dependent hybridization of primer P1 and P2 to a complementary sequence
(6) elongation of primer P1 and P2 using deoxyribonucleotides and a DNA-polymerase wherein synthesized DNA strands are complementary to cDNA second and first-strand;
(7) and elongation products are again target DNA for the steps (4), (5) and (6) which are repeated several times
(8) wherein said primer P1 and primer P2 are complementary (at least in parts) to the 1^{st}- and 2^{nd}-strand target nucleic acid.

Components of a RT-reaction typically include RNA-template, from which the complementary DNA (cDNA) is transcribed; a primer, which hybridizes to target nucleic acid; a nucleic acid polymerase that exhibits RNA-dependent DNA-polymerase activity; and the appropriate nucleotide building blocks needed for nucleic acid synthesis.
mixture is first incubated at a temperature sufficient to allow synthesis of a DNA molecule complementary to at least a portion of an RNA template. Components

### Isothermal amplification reaction

### Isothermal nucleic acid amplification (exponential):

Specific primed DNA-synthesis reactions for the use during *in-vitro* transcription based isothermal exponential nucleic acid amplification like NASBA (Nucleic Acid Sequence Based Amplification), 3SR (Self-Sustained Sequence Replication), 2SR (Self-Sustained Sequence Replication similar to 3SR), TMA (Transcription-mediated Amplification) and similar methods can be performed using sequence-specific oligonucleotide-primers, RNA-directed and DNA-directed DNA-polymerase and DNA-directed RNA-polymerase wherein said methods comprises the steps:
(1) providing a single reaction medium containing reagents comprising a target nucleic acid, a first oligonucleotide-primer P1, a second oligonucleotide-primer P2, an RNA-directed DNA polymerase, a DNA-directed DNA-polymerase, a DNA-directed RNA-polymerase, ribonucleotides and deoxyribonucleotides;
(2) providing conditions such that a amplification cycle is maintained wherein
(3) synthesis of a DNA first-strand complementary to the target nucleic acid is performed by hybridizing a oligonucleotide-primer P1 to a complementary sequence of the target nucleic acid followed by elongation of oligonucleotide-primer P1 using deoxyribonucleotides and a RNA-directed or DNA-directed DNA-polymerase, wherein said target nucleic acid is deoxyribonucleic acid (RNA) or ribonucleic acid (DNA);
(4) synthesis of a DNA second-strand complementary to the DNA first-strand is performed by enzymatic, chemical or thermal removal of the target nucleic acid from DNA first-strand followed by hybridizing a second oligonucleotide-primer P2 to a complementary sequence of the DNA first-strand followed by elongation of said oligonucleotide-primer P2 using deoxyribonucleotides and a DNA-directed DNA-polymerase wherein DNA second-strand is complementary to DNA first-strand;
(5) wherein said oligonucleotide-primer P1 bears a sequence at its 3'-ends hybridizing to the target nucleic acid (P1);
(6) wherein said oligonucleotide-primer P2 bears a sequence at its 3'-ends hybridizing to the first-strand DNA;
(7) wherein said oligonucleotide-primer P1 and/or oligonucleotide-primer P2 bears a DNA-directed RNA-polymerase promoter sequence at its 5'-end;
(8) transcribing *in-vitro* said DNA-template generated by the elongation of oligonucleotide-primer P1 and oligonucleotide-primer P2 (step 3 and 4) into RNA using DNA-directed RNA-polymerase and ribonucleotides.

### Isothermal nucleic acid amplification (linear)

Specific primed DNA-synthesis reactions for the use during *in-vitro* transcription based isothermal linear nucleic acid amplification can be performed using at least one sequence-specific oligonucleotide-primer, RNA-directed and DNA-directed DNA-polymerase and DNA-directed RNA-polymerase wherein said methods comprises the steps:
(1) providing a single reaction medium containing reagents comprising a target nucleic acid, an oligonucleotide-primer P1, a second oligonucleotide-primer P2, an RNA-directed DNA polymerase, a DNA-directed DNA-polymerase, a DNA-directed RNA-polymerase, ribonucleotides and deoxyribonucleotides;
(2) providing conditions such that no amplification cycle is maintained wherein
(3) synthesis of a DNA first-strand complementary to the target nucleic acid is performed by hybridizing a oligonucleotide-primer P1 to a complementary sequence of the target nucleic acid followed by elongation of oligonucleotide-primer P1 using deoxyribonucleotides and a RNA-directed or DNA-directed DNA-polymerase, wherein said target nucleic acid is deoxyribonucleic acid (RNA) or ribonucleic acid (DNA);
(4) synthesis of a DNA second-strand complementary to the DNA first-strand is performed by enzymatic, chemical or thermal removal of the target nucleic acid from DNA first-strand followed by hybridizing a second oligonucleotide-primer P2 to a complementary sequence of the DNA first-strand or by using a hairpin-loop of the DNA first-strand followed by elongation of said oligonucleotide-primer P2 or said hairpin-loop of the DNA first-strand using deoxyribonucleotides and a DNA-directed DNA-polymerase wherein DNA second-strand is complementary to DNA first-strand;
(5) wherein said oligonucleotide-primer P1 bear a sequence at its 3'-ends hybridizing to the target nucleic acid (PI);
(6) wherein said oligonucleotide-primer P2 bear a sequence at its 3'-ends hybridizing to the first-strand DNA;
(7) wherein said oligonucleotide-primer P1 or oligonucleotide-primer P1 and/or oligonucleotide-primer P2 bears a DNA-directed RNA-polymerase promoter sequence at its 5'-end;
(8) transcribing *in-vitro* said DNA-template generated by the elongation of oligonucleotide-primer P1 and oligonucleotide-primer P2 (step 3 and 4) into RNA using DNA-directed RNA-polymerase and ribonucleotides.

### The Present Invention:

Ribonucleic acid (RNA) is a substance synthesized biologically and synthetically. RNA serves many functions, including information molecule, reaction substrate, reaction catalyst, recognition element, structural element etc. However, for most analysis methods and functions concerning RNA itself, or RNA as an analysis tool, the purity of RNA is important. That means that *e.g*., other molecules which bind to the RNA are able to inhibit the analysis/function of/using the RNA molecule or destroy the structure of the RNA molecule. For this reason it is important to eliminate the inhibitory or destroying function of molecules binding to the RNA.

Moreover, stable secondary structures of RNA often results in reduced function and analysis quality. Secondary structure depends on the environment of the RNA molecule.

The present invention provides for the addition of ammonium sulfate (NH₄)₂SO₄ to RNA in solution to eliminate the inhibitory or destroying effect of molecules binding to the RNA to be taken as information molecule, reaction substrate, reaction catalyst, recognition element, structural element etc. Furthermore (NH₄)₂SO₄ solves secondary structures of RNA to make RNA more accessible to reactions and analysis.

In one aspect, the present invention is directed to the addition of (NH₄)₂SO₄ to an environment containing RNA, where in a preferred embodiment the final concentration is below 20 g/100 ml (1.51 M). The addition of (NH₄)₂SO₄ to the environment neutralizes the inhibitory effects of agents that bind to, or cleave, the RNA. Such agents include cationic detergents (*e.g*., CATRIMOX, and cetyltrimethylammonium bromide=CTAB. *See, e.g.,* European Patent Application EP 1031626 A1), proteins, ethidium bromide, SybrGreen™ dye, polyamines (*e.g*., spermine, spermidine, putresceine etc.), charged polysaccharides, glycoproteins, nucleophiles, bases etc. With the presence of (NH₄)₂SO₄ in the environment, the inhibitory or cleaving properties of agents binding to RNA do not interfere with the function and analysis of the environment or the RNA contained therein.

The following examples are illustrative of the present invention and are not to be construed as a limitation thereof.

### EXAMPLES

### EXAMPLE 1

### NEUTRALIZATION OF INHIBITORY EFFECTS OF RNA-SAMPLES CONTAINING CATIONIC DETERGENTS

### Cationic detergents can inhibit reactions with RNA samples

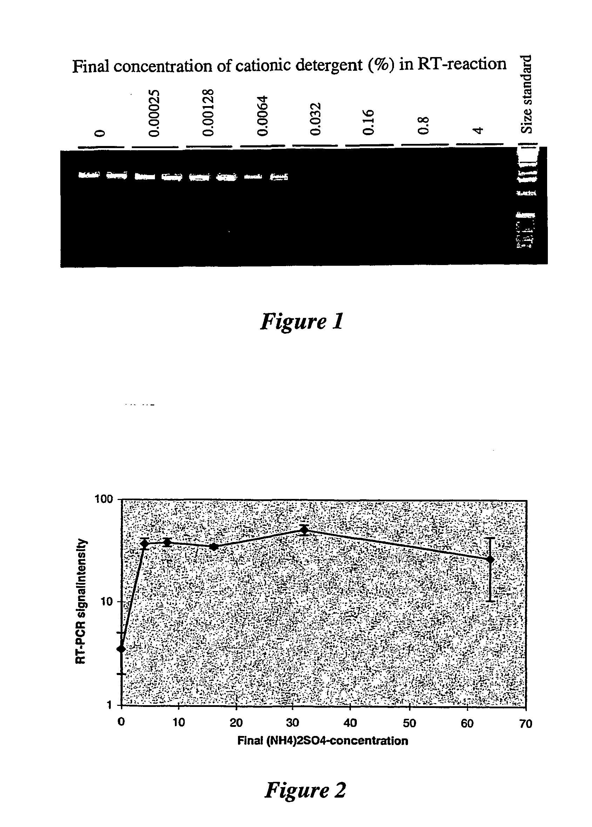

Cationic detergent at a final concentration of ≥ 0.0064 % is inhibitory during reverse transcription of RNA. This is shown in Figure 1.

### EXAMPLE 2

### ADDITION OF (NH₄)₂SO₄ TO RNA THAT INHIBITS REVERSE TRANSCRIPTASE REACTIONS DOES INCREASE THE PERFORMANCE OF REVERSE TRANSCRIPTION

Enzymatic reactions with RNA as template are crucial for cloning and expression analysis. Especially in reverse-transcriptase reactions (RT-reactions) the RNA is functionally and quantitatively analyzed.

Reverse transcriptase is not able to displace cationic detergents binding to RNA. RNA is masked and does not function as a template. Therefore the RNA cannot be analyzed quantitatively. Due to the binding of cationic detergents during reverse transcriptase polymerase chain reaction (RT-PCR) only very low signal intensities were obtained. A denaturation step (5 minutes at 65°C, shock cool on ice) of RNA does not solve the complex of RNA and cationic detergents. Addition of (NH₄)₂SO₄ to final concentration of 5-32 mM and denaturation of the sample for 5 minutes at 65°C with shock cool on ice does increase the RT-PCR signal significantly (Figure 2). The reverse transcriptase is not inhibited up to a final concentration of 32 mM (NH₄)₂SO₄.

### Experimental set-up:

Human blood RNA containing cationic detergent was dissolved in 10 µl water or in 10 µl of a (NH₄)₂SO₄ solution with a final concentration of 5-64 mM.The solution was denatured at 65°C for 5 minutes and cooled on ice. The whole solution was transferred to a 20 µl RT-reaction. RT-reaction was performed at 37°C. After RT-reaction was finished 2 µl of the RT-reaction was transferred to a 20 µl PCR. A 1700 bp fragment of the human ß-action sequence was detected with β-action specific primers.

### EXAMPLE 3

### THE ADDITION OF (NH₄)₂SO₄ TO THE RNA SAMPLE DOES ONLY INFLUENCE REACTIONS WITH RNA THAT IS INHIBITORY BUT NOT REACTIONS WITH RNA SAMPLES THAT IS NON-INHIBITORY

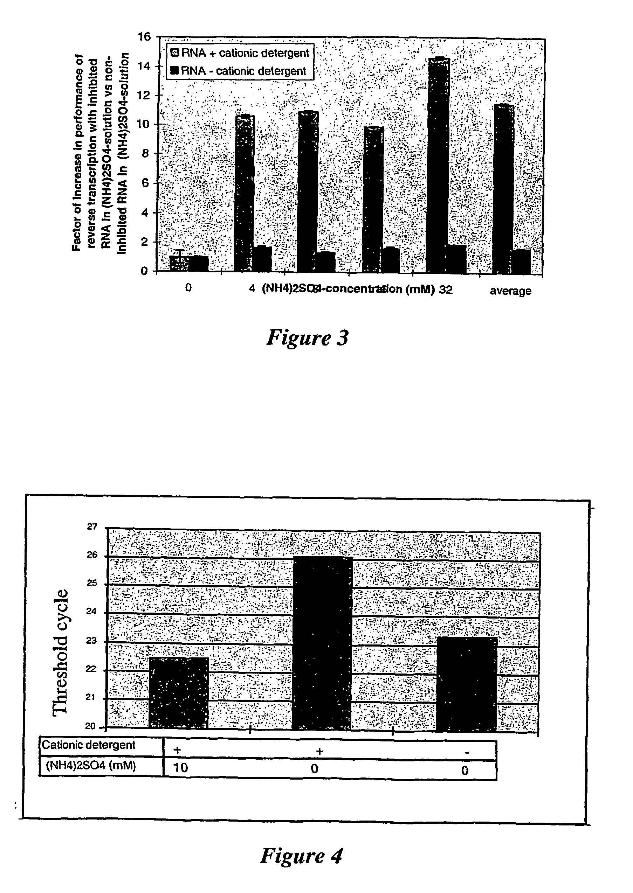

The addition of (NH₄)₂SO₄ to RNA that is inhibited by cationic detergents results in an about ≥ 10 fold increase in performance during reverse transcription. In contrast RNA that is not inhibited by cationic detergents showed only a very low increase (1.6 fold) in RT- reaction performance (Figure 3).

### Experimental set-up:

Total-RNA from Hela-cells containing cationic detergent (RNA+cationic detergent) was compared to total-RNA from Hela-cells containing no cationic detergent (RNA-cationic detergent). In order to determine the factor of increase because of the presence of different (NH₄)₂SO₄ concentrations RNA (with and without cationic detergents) was dissolved in 10 µl water or in 10 µl of a (NH₄)₂SO₄ solution with a final concentration of 5-30 mM. The solution was denaturated at 65°C for 5 minutes and cooled on ice. The whole solution was transferred to a 20 µl RT-reaction. RT-reaction was performed at 37°C. To determine the amount of synthesized cDNA, 2 µl of the RT-reaction was transferred to a 20 µl PCR and a 1700 bp fragment of the human ß-actin sequence was detected with ß-actin specific primers.

### EXAMPLE 4

### NEUTRALIZATION OF INHIBITORY EFFECTS OF RNA-SAMPLES CONTAINING CATIONIC DETERGENTS

### Addition of (NH₄)₂SO₄ to RNA that contain cationic detergents does increase the performance of TaqMan RT-PCR

TaqMan RT-PCR with RNA that contain cationic detergents results in bad performance (later threshold-cycle). Due to the binding of cationic detergents during reverse transcriptase polymerase chain reaction (RT-PCR) only high threshold-cycles were obtained. Addition of (NH₄)₂SO₄ to final concentration of 10 mM and denaturation of the sample for 5 minutes at 65°C with shock cool on ice does decreased the threshold cycle significantly (Figure 4).

### Experimental set-up:

Human blood RNA was prepared with cationic detergent or with a classical method without cationic detergent. The RNA was eluted with water, or with 10 mM (NH₄)₂SO₄. The eluate containing 10 mM (NH₄)₂SO₄ was denaturated at 65°C for 5 minutes and cooled on ice. An aliquot of each eluate was transferred to a single-tube TaqMan RT-PCR of GAPDH. Ingredients of the reaction are provided by Applied Biosystem (PDAR GAPDH).

### EXAMPLE 5

### NEUTRALIZATION OF INHIBITORY EFFECTS OF RNA-SAMPLES IS STABLE OVER TIME

### Addition of (NH₄)₂SO₄ to RNA that contain cationic detergents does increase the performance of TaqMan RT-PCR over time

The binding of cationic detergents to RNA can be neutralized with the addition of (NH₄)₂SO₄ over time. Only one initial denaturation (5 minutes at 65°C, shock cool on ice) is needed to neutralize the inhibitory effect of cationic detergent over time (at least over three weeks). No reassociation of cationic detergent and RNA is obtained in the presence of (NH₄)₂SO₄.

### Experimental set-up:

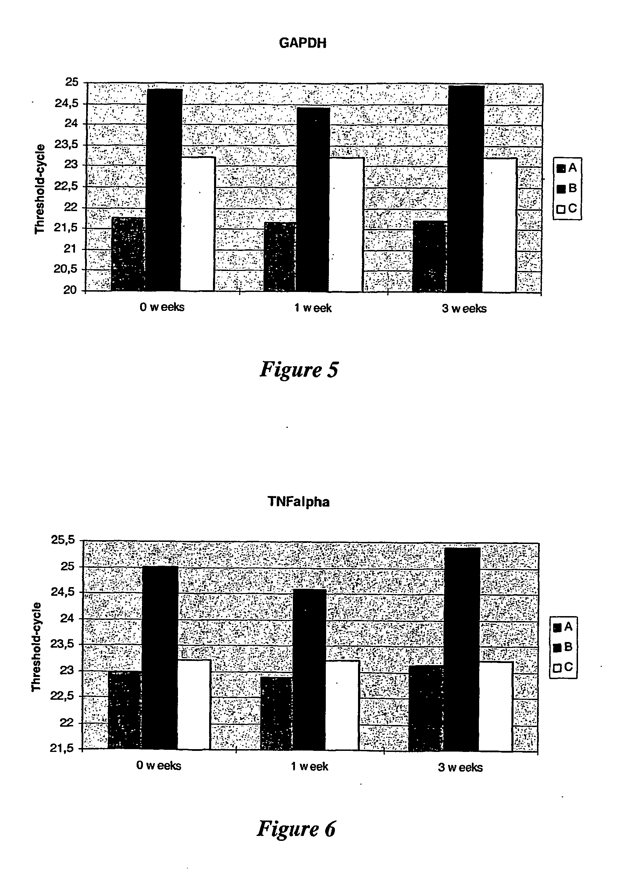

Human blood RNA was prepared with cationic detergent (A and B) or with a classical method without cationic detergent (C). The RNA was eluted with water (B and C) or with 10 mM (NH₄)₂SO₄ (A). The eluate containing 10 mM (NH₄)₂SO₄ was initially denaturated at 65°C for 5 minutes and cooled on ice. An aliquot of each eluate was transferred to a single-tube TaqMan RT-PCR of GAPDH (Figure 5) or TNFalpha (Figure 6). Other aliquots were stored at -20°C for 1 to 3 weeks. These aliquots were transferred to further TaqMan RT-PCR analyses without an additional denaturation step. The values are normalized to the threshold-cycle obtained with RNA prepared by the classical method (C).

### EXAMPLE 6

### NEUTRALIZATION OF INHIBITORY EFFECTS OF RNA-SAMPLES CONTAINING SPERMINE

### Spermine can inhibit reactions with RNA samples

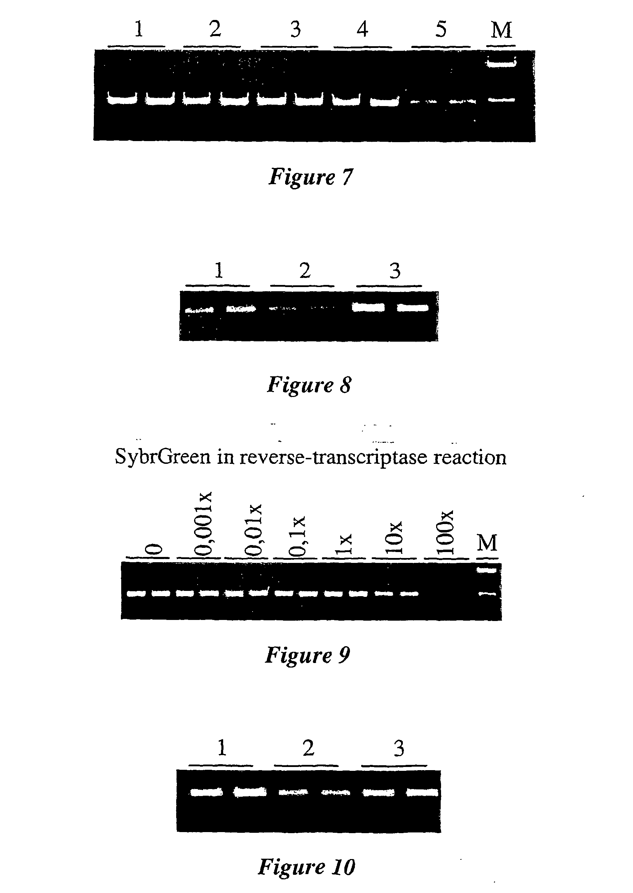

Spermine at a final concentration of 1 mM results in a up to 4 fold reduction during reverse transcriptase reaction (Figure 7).

### Experimental set-up:

Reverse-transcriptase reactions containing 0 mM (lanes 1), 0.125 mM (lanes 2), 0.25 mM (lanes 3), 0.5 mM (lanes 4) and 1 mM (lanes 5) spermine were performed. In order to quantify the generated cDNA after RT-reaction 2 µl of the RT-reaction was transferred to a 20 µl PCR. A specific fragment was analyzed by gel-electrophoresis (Figure 7).

### EXAMPLE 7

### ADDITION OF (NH₄)₂SO₄ TO RNA CONTAINING SPERMINE THAT INHIBITS REVERSE TRANSCRIPTASE REACTIONS DOES INCREASE THE PERFORMANCE OF REVERSE TRANSCRIPTION

Reverse transcriptase is not able to displace spermine binding to RNA. RNA is masked and does not function as a template. Therefore the RNA cannot be analyzed quantitatively. Due to the binding of spermine to RNA only very low signal intensities were obtained during reverse transcriptase polymerase chain reaction (RT-PCR). A denaturation step (5 minutes at 65°C, shock cool on ice) of RNA does not solve the complex of RNA and spermine. Addition of (NH₄)₂SO₄ to a final concentration of 5 mM and denaturation of the sample for 5 minutes at 65°C with shock cool on ice does increase the RT-PCR signal significantly (Figure 8).

### Experimental set-up:

Total RNA containing 5 mM spermine was dissolved in 2 µl water (lanes 2) or in 2 µl of a 5 mM (NH₄)₂SO₄ solution (lanes 1). In lane 3, total RNA without spermine was dissolved in 2 µl of a 5 mM (NH₄)₂SO₄ solution. The solution was denaturated at 65°C for 5 minutes and cooled on ice. The whole solution was transferred to a 20 µl RT-reaction. RT-reaction was performed at 37°C. After RT-reaction was finished 2 µl of the RT-reaction was transferred to a 20 µl PCR. A specific fragment was analyzed by gel-electrophoresis.

### EXAMPLE 8

### NEUTRALIZATION OF INHIBITORY EFFECTS OF RNA-SAMPLES

### CONTAINING SYBRGREEN™ DYE

### SybrGreen™ dye can inhibit reactions with RNA samples

SybrGreen™ dye at a final concentration of 100x results in a total loss of cDNA-synthesis (Figure 9).

### Experimental set-up:

Reverse-transcriptase reactions containing 0x, 0.001x, 0.01x, 0.1x, 1x, 10x and 100x SybrGreen™ dye were performed. In order to quantify the generated cDNA after RT-reaction, 2 µl of the RT-reaction was transferred to a 20 µl PCR. A specific fragment was analyzed by gel-electrophoresis.

### EXAMPLE 9

### ADDITION OF (NH₄)₂SO₄ TO RNA CONTAINING SYBRGREEN™ DYE THAT INHIBITS REVERSE TRANSCRIPTASE REACTIONS DOES INCREASE THE PERFORMANCE OF REVERSE TRANSCRIPTION

Reverse transcriptase is not able to displace SybrGreen™ dye binding to RNA. RNA is masked and does not function as a template. Therefore the RNA cannot be analyzed quantitatively. Due to the binding of SybrGreen™ dye to RNA, only very low signal intensities were obtained during reverse transcriptase polymerase chain reaction (RT-PCR). A denaturation step (5 minutes at 65°C, shock cool on ice) of RNA does not solve the complex of RNA and SybrGreen™ dye. Addition of (NH₄)₂SO₄ to final concentration of 5 mM and denaturation of the sample for 5 minutes at 65°C with shock cool on ice does increase the RT-PCR signal significantly (Figure 10).

### Experimental set-up:

Total RNA containing SybrGreen™ dye was dissolved in 2 µl water (lanes 2) or in 2 µl of a 5 mM (NH₄)₂SO₄ solution (lanes 1). In lanes 3, total RNA without SybrGreen™ dye was dissolved in 2 µl of a 5 mM (NH₄)₂SO₄ solution. The solution was denaturated at 65°C for 5 minutes and cooled on ice. The whole solution was transferred to a 20 µl RT-reaction. RT-reaction was performed at 37°C. After RT-reaction was finished 2 µl of the RT-reaction was transferred to a 20 µl PCR. A specific fragment was analyzed by gel-electrophoresis.

### EXAMPLE 10

### TIGHT JUNCTION OF (NH₄)₂SO₄ IONS TO RNA BACKBONE AND BASES

### Tight junction of (NH₄)₂SO₄ ions to RNA backbone results in competition and less electrophoretic mobility

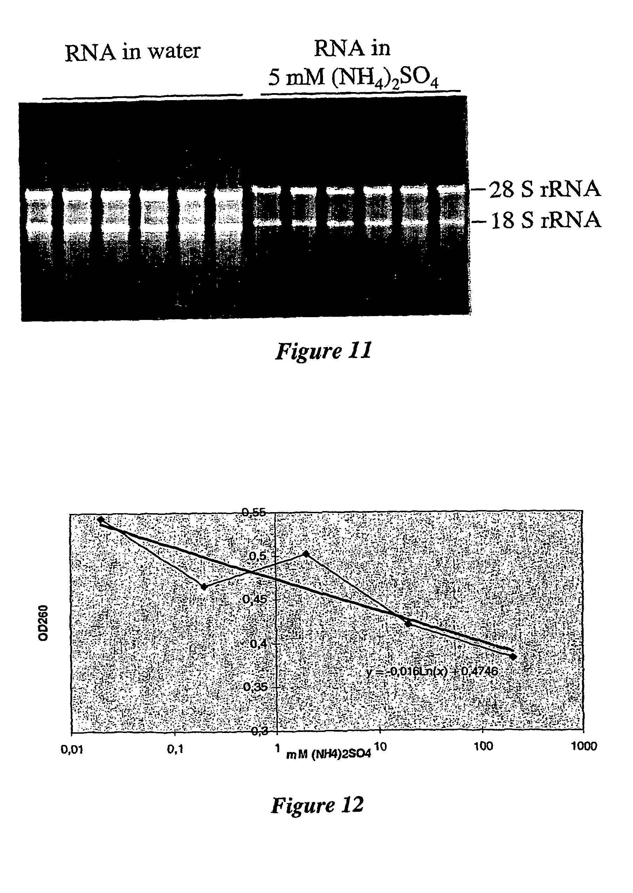

Ethidium bromide binds to the backbone of RNA as well as intercalates into double-stranded RNA. Under denaturating conditions (*e.g*., denaturating gel-electrophoresis) only single-stranded RNA does exist. Subsequently, under denaturating conditions, ethidium bromide binds to the RNA backbone via ionic interaction. This ionic interaction between ethidium bromide and RNA can be competed by the addition of (NH₄)₂SO₄ suggesting a tight interaction of (NH₄)₂SO₄ and RNA (Figure 11). This ethidium bromide displacement assay showed a tight junction between (NH₄)₂SO₄ and RNA.

### Experimental set-up:

2.6 µg total-RNA were dissolved in water or alternatively 2.3 µg total-RNA were dissolved in 5 mM (NH₄)₂SO₄. After addition of denaturating loading buffer containing formaldehyde and formamide the samples were denaturated at 65°C and loaded on a denaturating formaldehyde gel, as shown in Figure 11.

### EXAMPLE 11

The addition of (NH₄)₂SO₄ results in a retardation of electrophoretic mobility, suggesting a strong isolation of the negative charged RNA backbone by (NH₄)₂SO₄ (Figure 12). A further indication for the tight binding of (NH₄)₂SO₄ to RNA and its subsequent electric isolation came from the measurement of the hypochromic effect. The heterocyclic rings of the bases adsorb light at 260 nM. The hypochromic effect results from interactions between the electron systems of the bases made possible by their stacking in the parallel array of the double helix. Any decrease from the duplex state is immediately reflected by an increase in this effect - that means, by an increase in optical density at 260 nM (Figure 12).

### Experimental set-up:

RNA (22 µg/ml) is dissolved in different concentrations of (NH₄)₂SO₄. The optical density at 260 nM was measured, as shown in Figure 12.

### EXAMPLE 12

### TIGHT FUNCTION OF (NH₄)₂SO₄ IONS TO RNA BACKBONE RESULTS IN A BETTER STABILITY IN ENVIRONMENT CONTAINING NUCLEOPHILIC AGENTS

### Tight junction of (NH₄)₂SO₄ ions to RNA results in a better stability in alkali environment

RNA degrades rapidly in an environment of alkali pH due to basic hydrolysis by the nucleophilic OH⁻ ions. The addition of ammonium sulfate to the RNA in alkali environment stabilize the RNA and protects RNA from alkali hydrolysis.

### Experimental set-up:

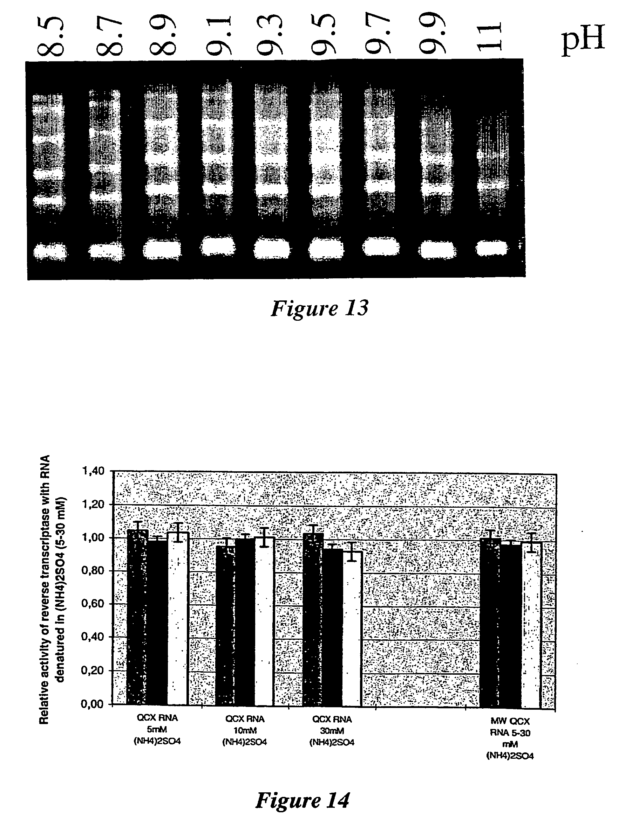

1 µg of 0.24-9.5 kb RNA-Iadder (LTI) was incubated for one hour at 37°C in a buffer containing 10 mM (NH₄)₂SO₄, 2 mM MgCl₂, 40 mM NaCl and 50 mM Tris-buffer at different pHs. (8.3 to 11). After addition of a denaturating gel-loading buffer the sample were denatured at 65°C and loaded on denaturating formaldehyde-gel. The gel is shown in Figure 13.

### EXAMPLE 13

### DOWN-STREAM ANALYSIS OF RNA DISSOLVED IN NH₄)₂SO₄ IS NOT AFFECTED

### The activity of Reverse Transcriptase is not affected by RNA-samples containing (NH₄)₂SO₄

Reverse Transcriptase is not affected by (NN₄)₂SO₄, as shown in Figure 14. Three different lots of reverse transcriptases were compared in activity assays in the absence or presence of ammonium sulfate in different concentrations. The activity of reverse transcriptases in the absence of ammonium sulfate was set to 1.0. (Figure 14).

### Experimental set-up:

A volume of 10 µl of total-RNA from Hela-cells in solutions with different (NH₄)₂SO₄ concentrations (0-30 mM) were spiked into RT-reactions. The relative activities compared to 10 µl of RNA in water were determined. All RNA-samples were denaturated at 65°C for 5 minutes and cooled on ice. The results are shown in Figure 14.

From the foregoing it will be appreciated that, although specific embodiments of the invention have been described herein for purposes of illustration, various modifications may be made without deviating from the spirit and scope of the invention.

### EXAMPLE 14

### ADDITION OF (NH₄)₂SO₄ TO RNA FOR LABELING REACTIONS WHICH PRODUCTS IS USED FOR MICRO-ARRAYS

Reverse transcriptase is not able to displace inhibitors tightly binding to RNA or tight base pairing of secondary structure. Therefore binding of inhibitors or stable secondary structures can affect analysis of transcripts by micro-arrays. A denaturation step of the RNA in a solution containing 5 mM (NH₄)₂SO₄ 5 minutes at 65°C with shock cool on ice does increase the number of positive signals and the signal-intensities on micro-arrays.

### Experimental set-up:

10 µg total-RNA was dissolved in 20 µl 5 mM (NH₄)₂SO₄ solution and was denatured at 65°C for 5 minutes. The solution was shock cooled on ice. In another experiment the RNA was not denatured in a (NH₄)₂SO₄ containing solution. Both RNAs were reverse transcribed by Omnscript Reverse Transcriptase using Cyanine-5-dCTP as a label. After the purification of the labeled cDNAs on QIAquick columns, the volume of cDNA-containing solutions were reduced by vacuum. The hybridizations of 1/5 of the purified cDNAs with micro-arrays were performed in a standard hybridization buffer over night. For washing of microarrays, standard washing buffers were used.

### Result:

With denaturation of RNA in a (NH₄)₂SO₄ containing solution prior labeling reaction more signals are visible and higher signal-intensities were obtained (B) compared to the standard labeling procedure without denaturation in a (NH4)₂SO₄ containing solution (A)

From the foregoing it will be appreciated that, although specific embodiments of the invention have been described herein for purposes of illustration, various modifications may be made without deviating from the spirit and scope of the invention.

## Claims

1. A method to neutralize the inhibitory effect of an agent that binds to, or cleaves RNA isolated from a natural source or artificially synthesized, comprising adding ammonium sulfate to a composition comprising said RNA and said agent, where the final concentration of ammonium sulfate in the composition is below 20 g / 100 ml.

2. The method of claim 1, where the final concentration of ammonium sulfate in the composition is about 1-64 mM.

3. The method of any one of claims 1-2, wherein the composition further comprises a contaminant selected from RNA binding agents.

4. The method of claim 1-2, wherein the composition further comprises a polyamine as a contaminant.

5. The method of claim 4, wherein the polyamine is selected from spermine, spermidine, and putresceine.

6. The method of any one of claims 1-5, wherein the composition further comprises cationic detergent.

7. The method of any one of claims 1-6, wherein the composition further comprises a nucleic acid dye as a contaminant.

8. The method of claim 7, wherein the nucleic acid dye is ethidium bromide or Sybr® Green.

9. The method of any one of claims 1-8, wherein the composition further comprises actinomycin.

10. The method of any one of claims 1-9, wherein the composition further comprises charged polysaccharide.

11. The method of any one of claims 1-10, wherein the composition further comprises glycoprotein.

12. The method of any one of claims 1-11, wherein the composition further comprises nucleophiles.

## Patentansprüche

1. Verfahren zur Aufhebung des inhibitorischen Effekts eines Agens, das an aus einer natürlichen Quelle isolierte oder künstlich synthetisierte RNA bindet oder diese spaltet, wobei man in dem Verfahren Ammoniumsulfat zu einer die RNA und das Agens umfassenden Zusammensetzung gibt, wobei die Endkonzentration von Ammoniumsulfat in der Zusammensetzung weniger als 20 g pro 100 ml beträgt.

2. Verfahren nach Anspruch 1, wobei die Endkonzentration von Ammoniumsulfat in der Zusammensetzung etwa 1-64 mM beträgt.

3. Verfahren nach einem der Ansprüche 1-2, wobei die Zusammensetzung ferner eine aus RNA bindenden Agentien ausgewählte Verunreinigung umfasst.

4. Verfahren nach Anspruch 1-2, wobei die Zusammensetzung ferner ein Polyamin als Verunreinigung umfasst.

5. Verfahren nach Anspruch 4, wobei das Polyamin aus Spermin, Spermidin und Putrescein ausgewählt ist.

6. Verfahren nach einem der Ansprüche 1-5, wobei die Zusammensetzung ferner kationisches Detergens umfasst.

7. Verfahren nach einem der Ansprüche 1-6, wobei die Zusammensetzung ferner einen Nukleinsäurefarbstoff als Verunreinigung umfasst.

8. Verfahren nach Anspruch 7, wobei es sich bei dem Nukleinsäurefarbstoff um Ethidiumbromid oder Sybr® Green handelt.

9. Verfahren nach einem der Ansprüche 1-8, wobei die Zusammensetzung ferner Actinomycin umfasst.

10. Verfahren nach einem der Ansprüche 1-9, wobei die Zusammensetzung ferner geladenes Polysaccharid umfasst.

11. Verfahren nach einem der Ansprüche 1-10, wobei die Zusammensetzung ferner Glycoprotein umfasst.

12. Verfahren nach einem der Ansprüche 1-11, wobei die Zusammensetzung ferner Nukleophile umfasst.

## Revendications

1. Procédé de neutralisation de l'effet inhibiteur d'un agent qui se lie à, ou clive, un ARN isolé d'une source naturelle ou synthétisé artificiellement, comprenant l'ajout de sulfate d'ammonium à une composition qui comprend ledit ARN et ledit agent, dans lequel la concentration finale de sulfate d'ammonium dans la composition est inférieure à 20 g/100 ml.

2. Procédé selon la revendication 1, dans lequel la concentration finale de sulfate d'ammonium dans la composition est d'environ 1 à 64 mM.

3. Procédé selon l'une quelconque des revendications 1 et 2, dans lequel la composition comprend en outre un contaminant choisi à partir de liants d'ARN.

4. Procédé selon les revendications 1 et 2, dans lequel la composition comprend en outre une polyamine comme contaminant.

5. Procédé selon la revendication 4, dans lequel la polyamine est choisie parmi la spermine, la spermidine et la putrescine.

6. Procédé selon l'une quelconque des revendications 1 à 5, dans lequel la composition comprend en outre un détergent cationique.

7. Procédé selon l'une quelconque des revendications 1 à 6, dans lequel la composition comprend en outre un colorant d'acide nucléique comme contaminant.

8. Procédé selon la revendication 7, dans lequel le colorant d'acide nucléique est du bromure d'éthidium ou du Sybr^{®}Green.

9. Procédé selon l'une quelconque des revendications 1 à 8, dans lequel la composition comprend en outre de l'actinomycine.

10. Procédé selon l'une quelconque des revendications 1 à 9, dans lequel la composition comprend en outre un polysaccharide chargé.

11. Procédé selon l'une quelconque des revendications 1 à 10, dans lequel la composition comprend en outre une glycoprotéine.

12. Procédé selon l'une quelconque des revendications 1 à 11, dans lequel la composition comprend en outre des nucléophiles.
